# EUROPEAN PATENT SPECIFICATION

(11) **EP 3 716 955 B1**
(45) Date of publication and mention of the grant of the patent: **13.03.2024**
(21) Application number: 18883349.5
(22) Date of filing: 26.11.2018
(51) Int. Cl.: A61K 9/48, A61K 9/00, A61K 31/05, A61K 31/352, A61K 45/06, A61J 1/14, A61J 1/03

(54) **SOFT DUAL CHAMBERED LIQUID-GEL CAPSULE AND METHOD TO DELIVER SUBLINGUAL AND INGESTIBLE CANNABIS COMPOSITIONS**
WEICHE DOPPELKAMMRIGE FLÜSSIGKEIT/GEL-KAPSEL UND VERFAHREN ZUR ABGABE VON SUBLINGUALEN UND UNVERDAULICHEN CANNABISZUSAMMENSETZUNGEN
CAPSULE DE GEL LIQUIDE À DOUBLE CHAMBRE SOUPLE ET PROCÉDÉ D'ADMINISTRATION DE COMPOSITIONS DE CANNABIS SUBLINGUALE ET INGÉRABLE

(30) Priority: 01.12.2017 US 201762593417 P
(43) Date of publication of application: 07.10.2020
(73) Proprietor: Healthy Option Consulting Inc., Boca Raton, FL 33498 (US)
(72) Inventor: FARBER, Andrew, Boca Raton, FL 33498 (US)
(74) Representative: ABG Intellectual Property Law, S.L.
(86) International application number: PCT/US2018/062421
(87) International publication number: WO 2019/108469

(56) References cited:
- EP-A1- 0 211 079
- WO-A1-2015/075221
- WO-A1-2016/029215
- WO-A1-2017/062954
- WO-A1-2017/145160
- WO-A2-02/064109
- KR-B1- 101 118 835
- TW-A- 200 924 806
- US-A1- 2015 274 344
- Kubota Rie ET AL: "Evaluation of the Method for Nifedipine Administration for a Rapid Onset of Clinical Effect: A Clinical Study in Normal Volunteers", YAKUGAKU ZASSHI, 1 January 2001 (2001-01-01), pages 355-364, XP055822960, Retrieved from the Internet: URL:https://yakushi.pharm.or.jp/FULL_TEXT/ 121_5/PDF/355.pdf [retrieved on 2021-07-09]
- SOLVAY PHARMACEUTICALS INC: "NDA 18-651/S-021; MARINOL (Dronabinol) capsules", INTERNET CITATION, 1 September 2004 (2004-09-01), pages 1-12, XP002671413, Retrieved from the Internet: URL:http://www.fda.gov/ohrms/dockets/docke ts/05n0479/05N-0479-emc0004-04.pd [retrieved on 2012-03-13]
- FRANCIOSI, A.: THC Strips: Everything You Need To Know About The Newest Cannabis Craze, 4 September 2017 (2017-09-04), XP055617234,
- MEAD, A.: "The Legal Status of Cannabis (Marijuana) and Cannabidiol (CBD) Under U.S. Law", Epilepsy & Behavior, vol. 70 4 February 2017 (2017-02-04), pages 288-291, XP085033473, DOI: doi:10.1016/j.yebeh.2016.11.021 Retrieved from the Internet: URL:http://dx.doi.org/10.1016/j.yebeh.2016 .11.021

## Description

### Field of the Invention.

The invention relates to a soft dual chambered liquid-gel capsule containing a sublingual cannabis composition in a first chamber and an ingestible cannabis composition in a second chamber, and said capsule for use in a method of administering the cannabis products to a patient in need of the cannabis product using the dual chambered liquid-gel capsule.

### Background of the invention.

Sublingually administered medical cannabis has rapid onset of action, and when administered in such a fashion can provide immediate relief for the patient. Sublingually administered medical cannabis is currently applied in the form of a bottled oil or tincture that is introduced into the body with a dropper and requires the patient or caregiver to administer the proper dose. Smoking and vaping medical cannabis also have rapid onset of action and can provide the patient with immediate relief.

Orally ingested medical cannabis is typically administered in the form of manufactured edibles or single chambered liquid-gel capsules infused with cannabis oil or pulverized cannabis flower containing cannabinoids of recommended strains, ratios and dosages. Due to the delayed onset of action of ingested medical cannabis, those not familiar with its effects often believe that they have not ingested enough due to this delayed onset of action, ingest more than they should, and suffer the nonlethal, yet ill effects of its excessive use.

Those currently seeking immediate and long term relief from the use of medical cannabis have limited options. They can manually combine: a) smoking and then ingesting; b) vaping and then ingesting; or c) sublingual application of bottled tinctures or oils and then ingesting. These methods are inconvenient and have the following additional shortcomings as described in the following paragraphs.

Sublingual application in the form of tinctures or oils with ingestion of edibles or infused capsules: Patient's seeking immediately relief or their caregivers are often stressed by their own or the patient's acute need, and must properly remove tincture or oil from the bottle and administer the correct dose. When using a dropper, especially when applied between the cheek and gum, it is likely that some of the introduced tincture or oil will be accidentally swallowed, thereby prevented immediate relief with respect to the amounts swallowed. Moreover, the process is inconvenient and can be unreliable in terms of assuring proper dosing.

Smoking with ingestion of edibles or infused capsules: The smoking of cannabis flower has been found to have adverse respiratory and other effects on humans. Not all jurisdictions permit the smoking of medical cannabis. Many patients, such as children, adolescents and the elderly cannot tolerate smoking medical cannabis and many simply do not want to introduce it into their bodies by smoking it. Even when using identical genetics, variations in the cannabis cultivation, drying and curing processes can adversely affect the uniformity of product and consistency of effects. Moreover, as inhalation of smoked flower differs from person to person, dosing can be uncertain.

Vaping with ingestion of edibles or infused capsules: The effects of vaping medical cannabis on humans have not been sufficiently investigated. Many patients, such as children, adolescents and the elderly cannot tolerate vaping medical cannabis and many simply do not want to introduce it into their systems by vaping it. Moreover, as inhalation of vaporized cannabis differs from person to person, dosing can be uncertain.

This leaves many medical cannabis patients with the inability to obtain quick, long lasting and accurately administered relief from medical cannabis that is administered in a manner with which they are comfortable.

Current liquid-gel capsules, including those that currently contain cannabis oils, are currently comprised of one chamber, do not provide for an immediate and extended release effect, and do not provide for the introduction of different compounds in one capsule. Liquid-gel capsules are sold in standard medicine bottles with a screw-on or other lid.

U.S. Patent No. 4,936,461 discloses a dual chamber capsule having a frangible dividing section. This patent does not disclose a gel capsule configured to provide sublingual and ingestible compositions.

WO 02/064109 discloses dosage forms for the mucosal administration of lipophilic medicaments, whereby the drug may optionally be hemp oil, and the dosage form may optionally be a crushable soft gel capsule.

### Summary of the Invention.

An objective of the invention is to provide a liquid-gel capsule that can efficiently provide multiple modalities of administration, including the application of both a sublingual cannabis composition and an ingestible cannabis composition.

Other objectives of the invention include providing a delivery system for immediate and long lasting effects, that is in a convenient capsule form, that reduces liquid mess, provides accuracy of dosing, avoids the risks of smoking/vaping, and eliminates the tendency to over medicate. Use of the term cannabis composition includes a singular cannabis composition and a plurality of cannabis compositions.

Other objectives of the invention include providing a delivery system for different cannabis products, each having different desired effects, where the timing of the delivery of the desired effects can be managed by the patient or the patient's caregiver.

These objectives and other objectives are obtained by a cannabis liquid-gel capsule for supplying a cannabis product to a patient in need of the cannabis product comprising:
a soft liquid-gel capsule comprising a first chamber and a second chamber; a sublingual cannabis composition disposed within the first chamber, the sublingual cannabis composition is formulated to be exposed within a mouth and under the tongue of a patient when the patient pierces the first chamber and pours the sublingual cannabis composition into the mouth and under the tongue, the first chamber being constructed to be pierceable and allow the sublingual cannabis composition to flow into the mouth and under the tongue; and
an ingestible cannabis composition disposed within the second chamber, the ingestible cannabis composition is formulated to be exposed and absorbed in the patient's gastrointestinal tract and the second chamber is constructed to dissolve or break apart in the stomach or small intestine to allow the ingestible cannabis composition to enter the lower gastrointestinal tract.

The objectives and other objectives can also be obtained by a lid for a medicine container constructed to contain soft liquid-gel capsules comprising an open cylinder formed on or connected to the underside of the container lid that is sized so that an end of the liquid-gel capsule can be inserted into the open cylinder, a piercing pin is located within the open cylinder so that when the liquid-gel capsule inserted into the cylinder the piercing pin pierces a chamber of the liquid-gel capsule, and the open cylinder is defined by a cylinder wall that is longer than the piercing pin so that a patient cannot contact the piercing pin. The lid is not claimed in the present invention.

These objectives and other objectives are obtained by a medicine bottle comprising a lid and containing at least one soft liquid-gel capsule, the lid comprising an open cylinder formed on or connected to a bottom of the container lid that is sized so that an end of the liquid-gel capsule can be inserted into the open cylinder, a piercing pin is located within the open cylinder so that when the liquid-gel capsule inserted into the cylinder the piercing pin pierces a chamber of the liquid-gel capsule, and the open cylinder is defined by a cylinder wall that is longer than the piercing pin so that a patient cannot contact the piercing pin. The medicine bottle is not claimed in the present invention.

These objectives and other objectives are obtained by a soft liquid-gel capsule comprising a first chamber and a second chamber, a sublingual cannabis composition disposed within the first chamber, and an ingestible cannabis composition disposed within the second chamber for use in medicine for supplying a cannabis product to a patient in need of the cannabis product; wherein the administration is carried out by
piercing the first chamber and flowing the sublingual cannabis composition into the patient's mouth and under the patient's tongue;
swallowing the second chamber; and
allowing the second chamber to dissolve or break open in the stomach or small intestine to release the oral cannabis composition.

Brief Description of the Drawings.

The following Detailed Description can be better understood when read in conjunction with the appended drawings. For the purposes of illustration, the drawings show example embodiments, but the subject matter is not limited to the specific elements and instrumentalities disclosed.
Fig. 1 illustrates a side view of a dual chamber liquid-gel capsule.
Fig. 2 illustrates a cut-away view of a dual chamber liquid-gel capsule.
Fig. 3 illustrates an end view of a dual chamber liquid-gel capsule.
Fig. 4 illustrates a bottom view of a container lid having a piercing chamber and piercing pin located on an inside of the bottle top (not part of the claimed invention).
Fig. 5 illustrates a side view of container lid having a piercing chamber and piercing pin located on an inside of the bottle top (not part of the claimed invention).
Fig. 6 illustrates a half liquid-gel capsule.
Fig. 7 illustrates a liquid-gel capsule.
Fig. 8 illustrates a half liquid-gel capsule.
Fig. 9 illustrates a half liquid-gel capsule.
Fig. 10 illustrates a liquid-gel capsule.

### Detailed Description of the Invention.

The invention will now be explained with reference to the attached nonlimiting figures.

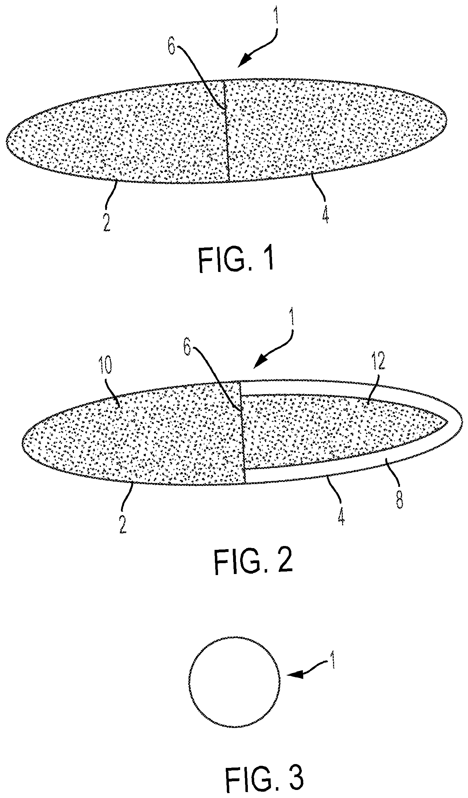

Figs. 1-3 illustrate an example of the present invention comprising a two chambered liquid-gel capsule 1, with each chamber being able to contain specifically dosed cannabis oil comprising cannabinoids recommended by the patient's physician or as otherwise predetermined. A first chamber 2 is constructed to be pierced, allowing the pre-dosed cannabis oil/cannabinoids 10 contained therein to be released sublingually into the mouth and under the tongue of the patient to provide for immediate onset of action. The pre-dosed cannabis oil/cannabinoids 10 are preferably liquid to facilitate flow from the first chamber 2 when pierced. A second chamber 4 is to be ingested orally by the patient and release the pre-dosed cannabis oil/cannabinoids 12 into the stomach or small intestine when the second chamber 4 decomposes or opens in the stomach or small intestine. In this manner, the present invention can provide immediate and long lasting relief. It can also provide for the administration of different types of cannabis products, each with desired effect, onset of action and duration of action.

Preferably, the first chamber 2 and second chamber 4, separated by the barrier 6, are identified 30 so that the proper first chamber 2 is pierced by the user, such as by providing an identifying mark or color 30 on the chamber to be pierced by a piercing device. The second chamber 4 that is not to be pierced can be made resistant to piercing by having a thicker or piercing resistant wall 8. The ingestible composition 12 can be viscous, semi-solid, or solid so that if the second chamber 4 having the ingestible composition 12 is wrongly pierced, the contents will substantially remain within the second chamber 4. The barrier 6 between the first and second chambers 2, 4 is not frangible and permanently holds the first and second chambers 2, 4 together. The gel capsule 1 can be formed using conventional methods, such as by forming two sections, filling the two sections with the compositions 10 and 12 and molding the two sections together.

There are soft liquid gel capsules, like Advil, and hard gel capsules. The invention relates to soft gelatin capsules that are configured to contain liquids. Soft liquid gel capsules usually comprise gelatin, a plasticizer and water. In contrast, hard gel capsules usually only contain gelatin and water, no plasticizer, to provide a hard shell. Hard gel capsules are usually preformed, shipped and then later filled. Soft liquid gel capsules are formed and then filled soon after forming the capsule or simultaneously with the formation of the capsule.

There are numerous types of medical marijuana formulations that are typically recommended, three examples of which are:
a) high tetrahydrocannabinol (THC) low cannabidiol (CBD) (of varying ratios)
b) high CBD low THC (of varying ratios)
c) roughly 1:1 balanced ratio THC/CBD

THC molecules have psychoactive effects while CBD and most other cannabinoid molecules do not. The presence of THC or THC content/psychoactive effect can be identified on the outside the capsule based on the color, mark, or other identifier 30 on one half of the gelcap.

If the medical condition requires extended amounts of THC the gel-capsule 1 would contain all of the first type of formulation with immediate onset of action (sublingual) in the first chamber 2 and delayed onset (ingested) in the second chamber 4. The gel-cap 1 can be sized as desired, for example to hold from 1 to 500 milligram (mg) of composition in each chamber 2, 4, preferably from 1 to 200 mg of composition in each chamber 2, 4.

On the other hand, some people can only tolerate high THC formulations at night or otherwise need it at night to combat insomnia. Therefore, it may be advantageous to have lower THC product released during the day and higher THC products actually delivered at night.

Sublingual cannabis compositions 10 for use in the first chamber 2, otherwise known as tinctures or oils, are well known. Any desired sublingual composition 10 can be utilized in the present invention. Sublingual cannabis compositions 10 are typically sold with a dropper for supplying drops of the composition to the mouth. The sublingual cannabis compositions can be pure cannabis oil. However, the sublingual cannabis composition 10 can include any carriers, additives, flavorings, solvents, stabilizers, preservatives, disbursing agents, or other agents as desired.

Ingestible cannabis compositions 12 for use in the in the second chamber 4 can be in any desired form since the ingestible cannabis composition is carried to the stomach by the liquid-gel capsule and introduced into the lower digestive tract. Thus, food or liquids are not necessary. The ingestible cannabis composition 12 can be a solid or liquid inside since it is contained in the second chamber 4 of the liquid-gel capsule 1. Once the liquid-gel capsule 1 reaches the stomach, the liquid-gel capsule 1 disintegrates or dissolves to allow the ingestible cannabis composition 12 to be consumed in the stomach and introduced into the lower digestive tract. The edible cannabis compositions 12 can be pure cannabis oil. However, the ingestible cannabis composition 12 can include any carriers, additives, flavorings, foods, stabilizers, preservatives, solvents, dispersing agents, or other agents as desired.

**Table 1 provides different exemplary compositions in the liquid-gel capsule 1.**

| SAME MEDICAMENT | | DIFFERENT MEDICAMENT | |
|---|---|---|---|
| First Chamber 2 | Second Chamber 4 | First Chamber 2 | Second Chamber 4 |
| Sublingual | Ingested | Sublingual | Ingested |
| Immediate Onset | Delayed Onset | Immediate Onset | Delayed Onset |
| | Extended | | Extended |
| Hi CBD-Lo THC | Hi CBD-Lo THC | Hi CBD-Lo THC | Hi THC-Lo CBD |
| Balanced | Balanced | Balanced | Hi THC-Lo CBD |
| CBD/THC | CBD/THC | CBD/THC | |
| Hi THC-Lo CBD | Hi THC-Lo CBD | Hi THC-Lo CBD | Balanced |
| | | | CBD/THC |

The cannabis composition comprises at least one cannabinoid.

The capsule is also useful for other lipophilic compounds and medications, but they do not form part of the claimed invention.

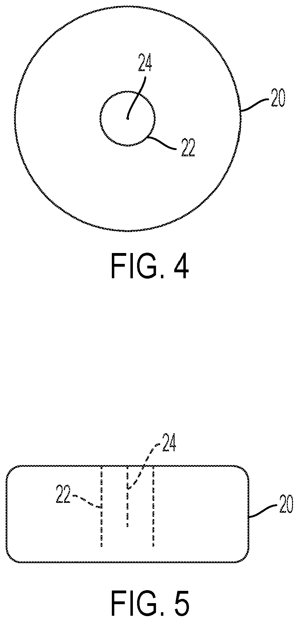

As shown in Figs. 4-5, further disclosed but not claimed is a gel-cap piercing device configured for piercing the first chamber 2 of the gel-cap so that the contents 10 can flow from the first chamber 2 by squeezing or sucking into the patient's mouth and under the tongue or between the cheek and gum. An example the gel-cap piercing device is a medicine bottle lid 20 having a piercing pin 24 located in an open cylinder 22 centered on the underside of the medicine bottle lid 20. The open cylinder 22 has an inner circumference just large enough to permit the insertion of the first chamber 2 of the liquid-gel capsule 1 into the open cylinder 22 so that the inserted end of the liquid-gel capsule 1, first chamber 2 can be pierced so that the contents 10 of the first chamber 2 can be sublingually or otherwise applied to the patient. The needle or piercing pin 24 is recessed in the open cylinder 22, i.e. the open cylinder 22 extends from the bottle lid 20 farther than the piercing pin 24, so that the piercing pin 24 cannot cause any physical harm to anyone handling the bottle lid 20. If desired, the liquid-gel capsule 1 can be constructed to be bitten to expose the contents 10 to the mouth.

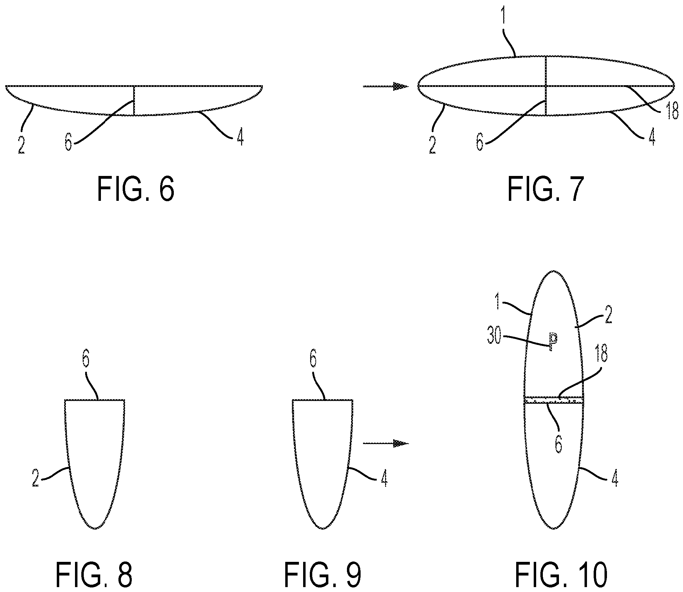

The production of liquid-gel capsules having one chamber is now well known. Conventional methods and materials used to form the liquid-gel capsules can be utilized to now form the dual chamber liquid-gel capsule 1. The prior art processes can be modified by one skilled in the art using the present invention to form a half gel capsule as shown in Fig. 6, like a canoe shape, in which each gel-cap half is half of each chamber 2 and 4 and half a barrier 6, fill each half of the gel-cap, and then bond two halves of the gel-cap together to form the liquid-gel capsule 1 as shown in Fig. 7 with a seal 18 bonding the two halves together. The two halves can be fused together using any conventional method, for example banding using a gelatin band or sealing using a hydroalcoholic solution to form the seal 18.

Alternatively, halves of the gel capsule as shown in Figs. 8 and 9, like a bullet shape, in which each half contains a whole chamber 2 or 4, can be formed. Each half of the gel-cap can be filled, and then bonded together at the barrier 6 to form the gel-cap 1 as shown in Fig. 10 with the seal 18 bonding the two halves together. The two halves can be fused together using any conventional method, for example banding using a gelatin band or sealing using a hydroalcoholic solution to form the seal 18.

The invention may also provide for the introduction of two different colored gelatins so that the consumer may identify and distinguish the contents contained in each chamber 2 and 4, as shown in Figs. 8 and 9, i.e. the half shown in Fig. 8 can be a different color than the half shown in Fig. 9. Different colored gel capsule materials may be used to generally identify high THC, high CBD or balanced products or may be used to distinguish between medicament intended for sublingual delivery and medicament intended to be ingested.

It should be understood, of course, that the foregoing relates to exemplary embodiments of the invention and that modifications may be made within the subject-matter of the claimed invention, which is set forth in the following claims.

## Claims

1. A cannabis liquid-gel capsule for supplying a cannabis product to a patient in need of the cannabis product comprising:
a soft liquid-gel capsule comprising a first chamber and a second chamber;
a sublingual cannabis composition disposed within the first chamber, the sublingual cannabis composition is formulated to be administered within a mouth and under the tongue of a patient when the patient pierces the first chamber and administers the sublingual cannabis composition into the mouth and under the tongue, the first chamber being constructed to be pierceable and allow the sublingual cannabis composition to flow into the mouth and under the tongue; and
an ingestible cannabis composition disposed within the second chamber, the ingestible cannabis composition is formulated to be administered and absorbed in the patient's gastrointestinal tract and the second chamber is constructed to dissolve or break apart in the stomach or small intestine to allow the ingestible cannabis composition to enter the lower gastrointestinal tract.

2. The cannabis liquid-gel capsule according to claim 1, wherein an identifier is present on liquid-gel capsule identifying the presence of THC product, THC and CBD product, CBD product, and/or psychoactive content.

3. The cannabis liquid-gel capsule according to claim 1, wherein a first identifier is present on liquid-gel capsule identifying a type of the sublingual cannabis composition and a second identifier is present on the liquid-gel capsule identifying a type of the ingestible cannabis composition.

4. A soft liquid-gel capsule comprising a first chamber and a second chamber, a sublingual cannabis composition disposed within the first chamber, and an ingestible cannabis composition disposed within the second chamber for use in medicine for supplying a cannabis product to a patient in need of the cannabis product;
wherein the administration is carried out by piercing the first chamber and flowing the sublingual cannabis composition into the patient's mouth and under the patient's tongue;
swallowing the second chamber; and
allowing the second chamber to dissolve or break open in the patient's stomach or small intestine to release the oral cannabis composition into the digestive tract.

5. The soft liquid-gel capsule for use according to claim 4, wherein the liquid-gel capsule is removed from a medicine bottle comprising a lid, the lid comprising an open cylinder formed on or connected to a bottom of the container lid that is sized so that an end of the liquid-gel capsule can be inserted into the open cylinder, a piercing pin is located within the open cylinder, and the open cylinder is defined by a cylinder wall that is longer than the piercing pin so that a patient cannot contact the piercing pin, the method further comprising inserting the liquid-gel capsule into the cylinder so that the piercing pin pierces a chamber of the liquid-gel capsule.

6. The soft liquid-gel capsule for use according to claim 4, wherein an identifier is present on liquid-gel capsule identifying the presence of THC product, THC/CBD product, CBD product, and/or psychoactive content.

7. The soft liquid-gel capsule for use according to claim 4, wherein a first identifier is present on liquid-gel capsule identifying a type of the sublingual cannabis composition and a second identifier is present on the liquid-gel capsule identifying a type of the ingestible cannabis composition.

8. The soft liquid-gel capsule for use according to claim 4, wherein the sublingual cannabis composition is sucked from the first chamber by the patient.

## Patentansprüche

1. Cannabis-Flüssiggel-Kapsel zur Abgabe eines Cannabisprodukts an einen Patienten, der das Cannabisprodukt benötigt, aufweisend:
eine weiche Flüssiggel-Kapsel, die eine erste Kammer und eine zweite Kammer aufweist;
eine sublinguale Cannabis-Zusammensetzung, die in der ersten Kammer angeordnet ist, wobei die sublinguale Cannabis-Zusammensetzung so formuliert ist, dass sie in einem Mund und unter der Zunge eines Patienten verabreicht wird, wenn der Patient die erste Kammer durchsticht und die sublinguale Cannabis-Zusammensetzung in den Mund und unter die Zunge verabreicht, wobei die erste Kammer so konstruiert ist, dass sie durchstochen werden kann und die sublinguale Cannabis-Zusammensetzung in den Mund und unter die Zunge fließen kann; und
eine einnehmbare Cannabis-Zusammensetzung, die in der zweiten Kammer angeordnet ist, wobei die einnehmbare Cannabis-Zusammensetzung so formuliert ist, dass sie im Magen-Darm-Trakt des Patienten verabreicht und absorbiert wird, und die zweite Kammer so konstruiert ist, dass sie sich im Magen oder Dünndarm auflöst oder auseinanderbricht, damit die einnehmbare Cannabis-Zusammensetzung in den unteren Magen-Darm-Trakt gelangen kann.

2. Cannabis-Flüssiggel-Kapsel nach Anspruch 1, wobei eine Kennung auf der Flüssiggel-Kapsel vorhanden ist, die das Vorhandensein eines THC-Produkts, eines THC- und CBD-Produkts, eines CBD-Produkts und/oder eines psychoaktiven Inhalts identifiziert.

3. Cannabis-Flüssiggel-Kapsel nach Anspruch 1, wobei eine erste Kennung auf der Flüssiggel-Kapsel vorhanden ist, die eine Art der sublingualen Cannabis-Zusammensetzung identifiziert, und eine zweite Kennung auf der Flüssiggel-Kapsel vorhanden ist, die eine Art der einnehmbaren Cannabis-Zusammensetzung identifiziert.

4. Weiche Flüssiggel-Kapsel aufweisend eine erste Kammer und eine zweite Kammer, eine in der ersten Kammer angeordnete sublinguale Cannabis-Zusammensetzung und eine in der zweiten Kammer angeordnete einnehmbare Cannabis-Zusammensetzung zur Verwendung in der Medizin zur Abgabe eines Cannabisprodukts an einen Patienten, der das Cannabisprodukt benötigt:
wobei die Abgabe durchgeführt wird mittels Durchstechen der ersten Kammer und Einströmen der sublingualen Cannabis-Zusammensetzung in den Mund des Patienten und unter die Zunge des Patienten;
Schlucken der zweiten Kammer; und
Zulassen, dass sich die zweite Kammer im Magen oder Dünndarm des Patienten auflöst oder aufbricht, um die orale Cannabis-Zusammensetzung in den Verdauungstrakt freizusetzen.

5. Weiche Flüssiggel-Kapsel zur Verwendung nach Anspruch 4, wobei die Flüssiggel-Kapsel aus einer Medikamentenflasche entfernt wird, die einen Deckel aufweist, wobei der Deckel einen offenen Zylinder aufweist, der an einem Boden des Behälterdeckels ausgebildet oder mit diesem verbunden ist und der so bemessen ist, dass ein Ende der Flüssiggel-Kapsel in den offenen Zylinder eingeführt werden kann, ein Durchstechstift innerhalb des offenen Zylinders angeordnet ist und der offene Zylinder durch eine Zylinderwand definiert ist, die länger als der Durchstechstift ist, so dass ein Patient den Durchstechstift nicht berühren kann, wobei das Verfahren ferner das Einführen der Flüssiggel-Kapsel in den Zylinder aufweist, so dass der Durchstechstift eine Kammer der Flüssiggel-Kapsel durchsticht.

6. Weiche Flüssiggel-Kapsel zur Verwendung nach Anspruch 4, wobei eine Kennung auf der Flüssiggel-Kapsel vorhanden ist, die das Vorhandensein eines THC-Produkts, eines THC/CBD-Produkts, eines CBD-Produkts und/oder eines psychoaktiven Inhalts identifiziert.

7. Weiche Flüssiggel-Kapsel zur Verwendung nach Anspruch 4, wobei eine erste Kennung auf der Flüssiggel-Kapsel vorhanden ist, die eine Art der sublingualen Cannabis-Zusammensetzung identifiziert, und eine zweite Kennung auf der Flüssiggel-Kapsel vorhanden ist, die eine Art der einnehmbaren Cannabis-Zusammensetzung identifiziert.

8. Weiche Flüssiggel-Kapsel zur Verwendung nach Anspruch 4, wobei die sublinguale Cannabis-Zusammensetzung vom Patienten aus der ersten Kammer gesaugt wird.

## Revendications

1. Une capsule de gel liquide à base de cannabis pour fournir un produit à base de cannabis à un patient ayant besoin du produit à base de cannabis, comprenant :
une capsule de gel liquide souple comprenant une première chambre et une deuxième chambre ;
une composition de cannabis sublinguale disposée à l'intérieur de la première chambre, la composition de cannabis sublinguale étant formulée pour être administrée dans la bouche et sous la langue d'un patient lorsque le patient perce la première chambre et administre la composition de cannabis sublinguale dans la bouche et sous la langue, la première chambre étant construite pour être perçable et permettre à la composition de cannabis sublinguale de s'écouler dans la bouche et sous la langue ; et
une composition de cannabis ingérable disposée à l'intérieur de la deuxième chambre, la composition de cannabis ingérable est formulée pour être administrée et absorbée dans le tractus gastro-intestinal du patient et la deuxième chambre est construite pour se dissoudre ou se briser dans l'estomac ou l'intestin grêle pour permettre à la composition de cannabis ingérable de pénétrer dans le tractus gastro-intestinal inférieur.

2. La capsule de gel liquide de cannabis selon la revendication 1, dans laquelle un identifiant est présent sur la capsule de gel liquide identifiant la présence d'un produit THC, d'un produit THC et CBD, d'un produit CBD et/ou d'un contenu psychoactif.

3. La capsule de gel liquide de cannabis selon la revendication 1, dans laquelle un premier identifiant est présent sur la capsule de gel liquide identifiant un type de composition de cannabis sublinguale et un deuxième identifiant est présent sur la capsule de gel liquide identifiant un type de composition de cannabis ingérable.

4. Une capsule souple de gel liquide comprenant une première chambre et une deuxième chambre, une composition de cannabis sublinguale disposée à l'intérieur de la première chambre, et une composition de cannabis ingérable disposée à l'intérieur de la deuxième chambre pour une utilisation en médecine pour fournir un produit à base de cannabis à un patient ayant besoin du produit à base de cannabis ;
l'administration étant effectuée en perçant la première chambre et en faisant couler la composition de cannabis sublinguale dans la bouche du patient et sous la langue du patient ;
en avalant la deuxième chambre ; et
en permettant à la deuxième chambre de se dissoudre ou de s'ouvrir dans l'estomac ou l'intestin grêle du patient pour libérer la composition orale de cannabis dans le tube digestif.

5. La capsule souple de gel liquide à utiliser selon la revendication 4, dans laquelle la capsule de gel liquide est retirée d'un flacon de médicament comprenant un couvercle, le couvercle comprenant un cylindre ouvert formé sur un fond du couvercle du récipient, ou relié à celui-ci, qui est dimensionné de telle sorte qu'une extrémité de la capsule de gel liquide puisse être insérée dans le cylindre ouvert, une tige de perçage est située à l'intérieur du cylindre ouvert, et le cylindre ouvert est défini par une paroi de cylindre qui est plus longue que la tige de perçage de sorte qu'un patient ne peut pas entrer en contact avec la tige de perçage, le procédé comprenant en outre le fait d'insérer la capsule de gel liquide dans le cylindre de telle sorte que la tige de perçage perce une chambre de la capsule de gel liquide.

6. La capsule de gel liquide souple à utiliser selon la revendication 4, dans laquelle un identifiant est présent sur la capsule de gel liquide identifiant la présence d'un produit THC, d'un produit THC/CBO, d'un produit CBD et/ou d'un contenu psychoactif.

7. La capsule souple de gel liquide à utiliser selon la revendication 4, dans laquelle un premier identifiant est présent sur la capsule de gel liquide identifiant un type de composition de cannabis sublinguale et un deuxième identifiant est présent sur la capsule de gel liquide identifiant un type de composition de cannabis ingérable.

8. La capsule souple de gel liquide à utiliser selon la revendication 4, dans laquelle la composition de cannabis sublinguale est aspirée depuis la première chambre par le patient.
